Europäisches Patentamt

European Patent Office

Office européen des brevets

(11) Publication number: **0 267 697**
**A2**

# EUROPEAN PATENT APPLICATION

(21) Application number: **87309148.2**

(22) Date of filing: **15.10.87**

(51) Int. Cl.⁴: **B01D 50/00 , B01D 46/12 , B01D 53/14 , A61L 9/00 , A61L 9/14**

(30) Priority: 15.10.86 JP 244834/86
14.05.87 JP 72329/87
18.05.87 JP 74183/87
18.05.87 JP 74184/87

(43) Date of publication of application:
18.05.88 Bulletin 88/20

(84) Designated Contracting States:
**DE ES FR GB IT**

(71) Applicant: **MITSUBISHI DENKI KABUSHIKI KAISHA**
**2-3, Marunouchi 2-chome Chiyoda-ku**
**Tokyo 100(JP)**

(72) Inventor: **Kajiwara, Yasuo**
**Mitsubishi Denki K.K. Shizuoka Seisakusho**
**18-1**
**Ojika 3-chome Shizuoka-shi Shizuoka**
**422(JP)**
Inventor: **Izaki, Katsura**
**Mitsubishi Denki K.K. Shizuoka Seisakusho**
**18-1**
**Ojika 3-chome Shizuoka-shi Shizuoka**
**422(JP)**
Inventor: **Takatsuka, Youichi c/o Ryoden**
**Engineering K.K.**
**Nagoya Office Shizuoka Branch 18-1**
**Ojika 3-chome Shizuoka-shi Shizuoka**
**422(JP)**

(74) Representative: **Lawson, David Glynne et al**
**MARKS & CLERK 57-60 Lincoln's Inn Fields**
**London WC2A 3LS(GB)**

(54) **Air cleaner.**

(57) An air cleaner has a housing containing a dust filter 7 and a deodorizing filter 8, through which air is passed by a fan 1. To maintain the deodorizing effect, without being affected by the amount of dust collected on the filters, liquid deodorant is periodically applied to the deodorizing filter 8, from a perforated pipe 16 above the filter.

FIG. 2.

## AIR CLEANER

### Background of the Invention

This invention relates to an air cleaner having both dust collecting function and deodorizing function.

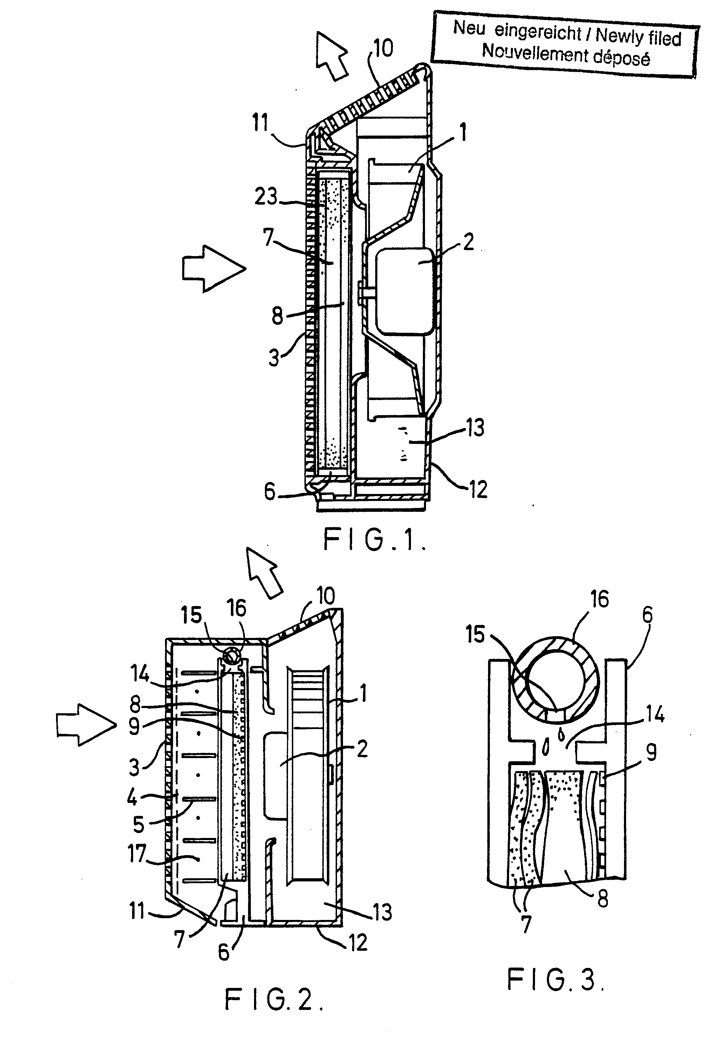

Fig. 1 is a cross sectional view illustrating a conventional air cleaner, for example, disclosed in Japanese Patent Laid-Open No. 87823/1985. In this drawing, reference numeral 1 represents a sirocco fan, and reference numeral 2 represents a fan motor. Reference numeral 3 represents a intaking grille which is provided for a front panel 11, reference numeral 10 represents an outlet port, reference numeral 7 represents a dust collecting filter, and reference numerals 8 and 23 represent deodorizing filters. These filters are laminated and accommodated in a filter frame 6 in such a manner that these filters are detachably secured upstream of the device.

The function of it will now be described. Air is taken in by a fan 1 which is driven by the fan motor 2 through the inlet grille 3 of a device casing 13. Dust particles in air are absorbed and caught, and offensive odor substances are simultaneously removed when the air then passes the group of the filters accommodated in the filter frame 6, and clean air is discharged through the outlet grille 10.

Fig. 1 illustrates a filter filtration type air cleaning device. However electrical air filters which are capable of collecting smaller dust are known in U.S. Patent Serial No. 288651 and Japanese Patent Publication No. 8913/1979 issued on April 19, 1979.

These electrical air filters remove dust in a similar deodorizing principle to the former in which a deodorizing filter impregnated with activated carbon or deodorant and dried is provided for a part of a laminated filter, whereby offensive odor substance is removed from the air which is passing through.

Since the conventional air cleaner is formed as mentioned above, the group of filters become foul and the surface of the deodorizing filter is covered with soil as the air filter is operated. Consequently, the deodorizing substance are gradually brought in less contact with the air including offensive odor, so that the deodorizing effect which is displayed when the filters are new rapidly deteriorates. Even if the dust collecting filter shows further capability of collecting dust, the whole body of the deodorizing filter is needed to be replaced with a new one. As a result there has been drawbacks in that the replacement is troublesome and is costly.

An object of this invention is to overcome these problems and to maintain deodorizing activity substantially unaffected by accumulated dirt.

According to the invention, a deodorant liquid is applied periodically (or even continuously) to the deodorizing filter. By this measure, deodorizing activity is maintained and filter changes can be reduced.

More specifically the invention provides an air cleaner comprising a dust collecting filter, a deodorizing filter and a fan for generating a flow of air through the filters, characterised in that the air cleaner is provided with a source of deodorant liquid, and means for distributing deodorant liquid from the source over the deodarizing filter.

In a preferred embodiment the air cleaner not only collects dust in air by means of a dust collecting filter, but is also optionally or periodically sprayed with deodorant through a spraying pipe thereof by the use of a pump. The filter can be supplied with deodorant by means of a pump at any time when it is required, when offensive odor is requried to be removed, when deodrizing effect of the filter is about to decline, and when the deodorizing effect is needed to be improved. Therefore, it is possible to obtain an air cleaner which is capable of providing a superior deodorizing effect which is not affected by the state of dust collection on the filter without requiring trouble in maintenace and much cost. deodorizing effect can be kept at low labor and cost.

### Brief Description of the Drawings

Fig. 1 is a cross sectional view illustrating a conventional air cleaner;

Fig. 2 is a cross sectional view illustrating an air cleaner of an embodiment according to the present invention;

Fig. 3 is an enlarged cross sectional view illustraing a deodorant spraying portion in Fig. 2;

Fig. 4 is a schematic view illustrating a deodorant supplying system in Fig. 2;

Fig. 5 is a schematic view illustrating a control system of the apparatus shown in Fig. 2;

Fig. 6 is a flow chart of the control system shown in Fig. 5;

Figs. 7 and 8 are views illustrating a deodorizing filter to which a seam is provided;

Fig. 9 is a cross sectional view illustrating a spraying pipe;

Fig. 10 is a graph showing the relationship between a spraying hole of a spraying pipe and an amount of deodorant fluid sprayed;

Fig. 11 is a side cross sectional view illustrating a connecting portion between a spraying pump and a deodorant chamber;

Fig. 12 is a perspective view illustrating the state in which the connecting portion shown in Fig. 11 is removed;

Fig. 13 is a side cross sectonal view illustrating an improved connecting portion beween a spraying pump and a deodorant chamber;

Figs. 14 is a perspective view illustrating the state wherein the connecting portion in Fig. 13 is removed;

Fig. 15 is a side cross sectional view illustrating an improved connecting portion;

Fig. 16 is a perspective view illustrating the state in which the connecting portion in Fig. 15 is removed;

Fig. 17 is a cutway view illustrating an essential portion of a deodorant chamber and a casing accommodating portion;

Fig. 18 is cross sectional view illustrating an essential portion in which a deodorant chamber is accommodated in the casing shown in Fig. 17;

Fig. 19 is a perspective view illustrating the deodorant chamber shown in Fig. 17; and

Fig. 20 is a cross sectional view illustrating other examles of the deodorant chamber and the casing accommodating portion.

## Description of the Preferred Embodiment

Referring to the accompanying drawings, an embodiment of an air cleaner will now be described.

In Fig. 2, reference numeral 1 represents a sirocco fan, reference numeral 2 represents a fan motor, and reference numeral 3 represents an intake grille which is disposed on a front surface of a front panel 11. Reference numeral 4 represents a prefilter which removes a relatively large sized-dust in the air taken in, and reference numeral 5 represents a discharge side grounding electrode. Reference numeral 6 represents a filter frame in which a dust collecting filter 7, a filter 8 used exclusively for deodorizing, and a dust collecting side grounding electrode 9 are accommodated and detachably mounted. Reference numeral 13 represents a casing which is disposed in a backpanel 12, and reference numeral 10 represents an outlet hole. Reference numeral 14 represents a deodorant guide which is disposed in the filter frame 6, and reference numeral 16 represents a deodorant spraying pipe having a plurality of spraying holes 15. Reference numeral 17 represents a high tension discharging cable.

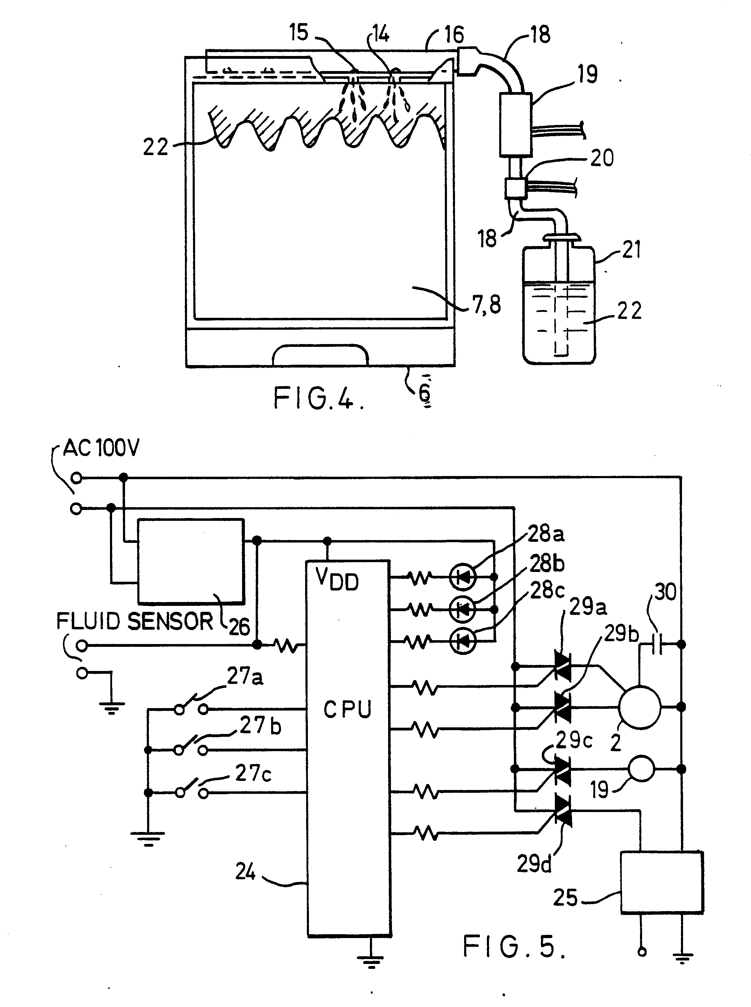

Fig. 3 is an enlarged cross sectional view illustrating a deodorant spraying portion. Fig. 4 is a -

schematic view illustrating a system of supplying deodorant, in which reference numeral 19 represents a spraying pump, reference numeral 18 represents a connecting pipe, reference numeral 20 represents a fluid sensor, reference numeral 21 represents a deodorant chamber and reference numeral 22 represents deodorant. Fig. 5 is a - schematic view illustrating the control of the device shown in Fig. 2, in which reference numeral 24 represents a CPU, reference numeral 25 represents a high power source unit, reference numeral 26 represents a power source circuit for a microcomputer, reference numerals 27a, 27b and 27c represent switches in which reference numeral 27c represents a deodorizing switch. Reference numerals 28a, 28b and 28c represent LEDs, reference numerals 29a, 29b, 29c and 29d represent double-throw thyristor switches, and reference numeral 30 represents a capacitor.

The operation of the air cleaner shown in Figs. 2 to 5 will now be described.

The fan 1 is rotated by the fan motor 2 in the device casing 13, whereby air is taken in through the intake grille. When passing through an ionized zone formed by the discharging cable 17 and the discharging side grounding plate 5, particles in air is charged in the device. Dust is absorbed by way of the reversed polarity charge to the charged particles, induced by a group of filters surrounded by a strong electrical field formed by the discharging cable 17 and the dust collecting side grounding electrode 9. The offensive odor substances are removed when they pass through the deodorizing filter 8. The operation mentioned above is the same as that of the conventional air cleaner.

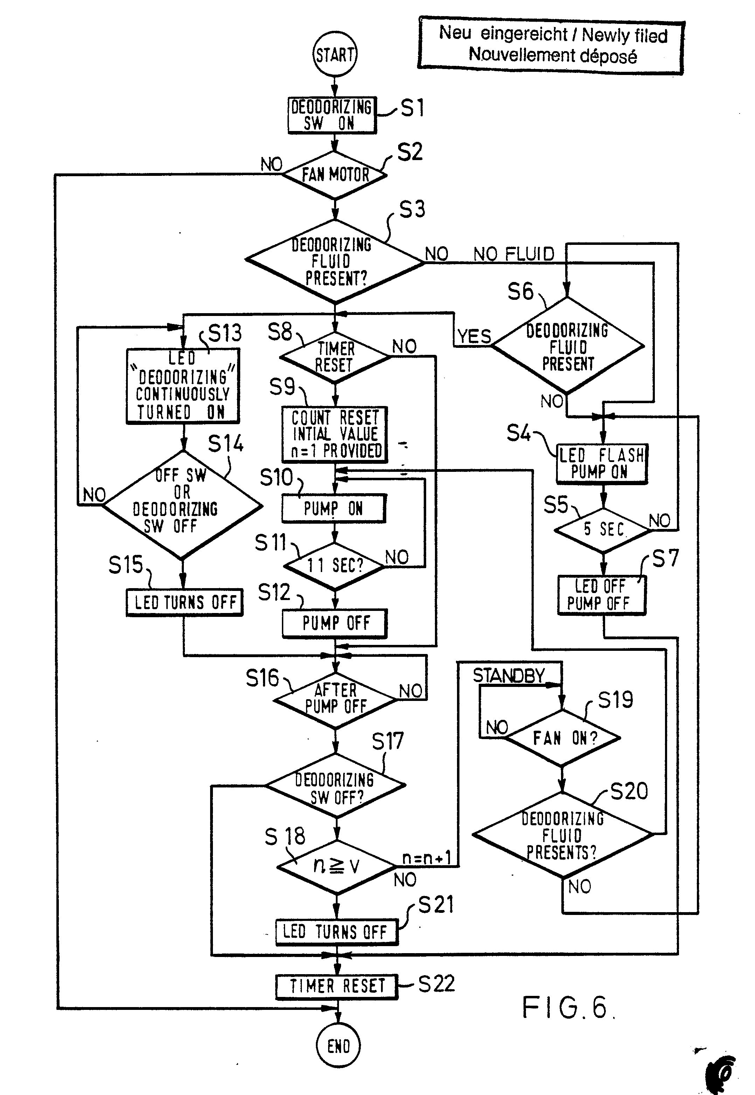

The deodorizing mechanism of an air cleaner according to the present invention has a mechanism in which deodorant 22 disposed in the device is supplied to the deodorizing filter 8. When the deodorizing switch 27c is pushed, the double-throw thyristor switch 29c which is controlled by the CPU 24 disposed in a control device is turned on, whereby the spraying pump 19 actuates. The deodorant enclosed in the deodorant chamber 21 is sucked and fed to the spraying pipe 16 through the connecting pipe 18. The deodorant 22 is delivered through a plurality of spraying holes 15 which are disposed in the spraying pipe 16. The deodorant is then applied to the deodorizing filter 8 through the deodorant guide 14 of the fitler frame 6. These successive operations for supplying the deodorant 22 are controlled by the CPU 24, as described in a flow chart shown in Fig. 6. Each step of this flow chart will now be described. The deodorizing switch 27c is turned on in step S1. Then in step S2, judgement is made as to whether or not the motor fan 2 has been turned on, if not, the operation proceeds to the final step where it is completed. If the motor fan 2 is turned on, it is then shifted to step S3 in which whether or not the deodorant is in the connecting pipe 18 which is connected to the deodorant chamber 21 is judged. If the state in which the deodorant 22 lacks is detected by the fluid sensor 20, it is then shifted to step S4. In this step, the LED 28 is flashed showing the lack of deodorant, and spraying pump 19 is turned on so as to introduce the deodorant 22 in the deodorant chamber 21 into the connecting pipe 18.

In step S6, judgement is made as to whether or not the deodorant 22 is detected by the fluid sensor 20, as a result of turning on of the spraying pump 19, and confirmation thereof is carried out in step S5 for a predetermined time, for examle, five sec. If the deodorant fluid 22 is not detected in the predetermined time, it is then shifted to step S7 in which the flashing of the LED 28 which has been started in step S4 is stopped, the spraying pump 19 is turned off, and the operation is brought into the timer reset state in step S22 in which the supply of the deodorant 22 into the deodorant chamber 21 is waited for.

If the presence of the deodorant 22 is detected in step S3 or step S6, it is shifted to both steps S8 and S13. In step S13, the LED 28 is continuously lit showing the deodorizing operation on, and then in setp S14, whether the electric source switch is turned off or not, or the deodorizing switch 27c is turned off or not is judged, if both of them are turned on, the LED 28 is continued to light in step S13. When either one of the electric source switch and the deodorizing switch 27c is turned off, the LED 28 is turned off in step S15, and it is then shifted to step S16.

Incidently, in step S8, whether the timer is reset or not is judged, and if the timer is not reset, it is then shifted to step S16.

When the timer is reset, it is then shifted to step S9, and the count is set to the initial value n = 1, and in the next step S10, the spraying pump 19 is turned on whereby the deodorant 22 is sprayed through the spraying hole 5, as a result of which the supply of the deodorant 22 to the deodorizing filter 18 is started.

Then, in step S11, whether time $t_1$ sufficient to allow the deodorizing filter 8 to be supplied with a sufficient quantity of the deodorant 22 has lapsed or not is judged.

Within time $t_1$ after turning on of the spraying pump 19, the spraying pump 19 is continued to operate in step 10. After an elapse of the time $t_1$, the spraying pump 19 is turned off in step S12.

In the next step S16, after turning off of the spraying pump 19, namely, once the deodorizing filter 8 is supplied with the deodorant 22, whether the time $t_2$ has lapsed in which the deodorant 22 is needed to be applied to the deodorizing filter 8 after the deodorant 22 acts on the deodorizing filter 8 or not is judged.

If the time $t_2$ has passed, it is then shifted to the next step S17 in which whether the deodorizing switch 27c is kept to turn on or not is judged. Then, in step S18, whether the numbers of current deodorizing operation n reaches the maximum numbers N which is the maximum number of the deodorizing operation after turning on of the deodorizing switch 27c withot any turning off is judged.

When it becomes $n \geq N$, it is then shifted to step S21 in which the LED 28 is turned off showing the series of deodorant 22 supplying function has been completed, then it is shifted to step S22.

If $n < N$ in step 18, n + 1 is set as new n, namely, the count is added by one, it is then shifted to step S19.

In step S19, whether the fan motor 2 which has been turned on in step S2 is kept to operate or not is judged.

If the fan motor 2 is turned off, waiting is required till the fan motor 2 is turned on in step S19.

If the fan motor 2 is turned on, it is then shifted to step S20 in which whether the deodorant 22 which is to be supplied next time is present or not is judged.

If the deodorant 22 is present in this step S20, it is then returned to the step S10 in which the spraying pump 19 is turned on, whereby aforesaid series of deodorant supplying operation is performed.

In this step S20, if the deodorant 22 is not detected by the fluid sensor 20, it is then shifted to step S4 in which the LED 28 is flashed showing the lack of the deodorant 22 and the spraying pump 19 is turned on.

That is, the pump 19 is turned on only in the case wherein the fan 1 of the air cleaner is rotated for the purpose of drying the deodorant, that is in the operation state of the air cleaner. Each spraying time is regulated so as to prevent overflow of the deodorant through the filter 8, and when the spraying is repeated at a proper interval and a predetermined numbers of spraying ( n times) is completed, the deodorant supplying operation pattern is adapted to automatically stop.

Furthermore, when the deodorant lacks, the LED flashes for alarming by way of detection by the fluid sensor 29. And this deodorant supplying operation and deodorant supplying operation are adapted to be optionally stopped, and if they are turned on again, the time from the previous spraying of the deodorant is always observed by the CPU 24 for the purpose of preventing overflow of the deodorant out of the device or dropping of it.

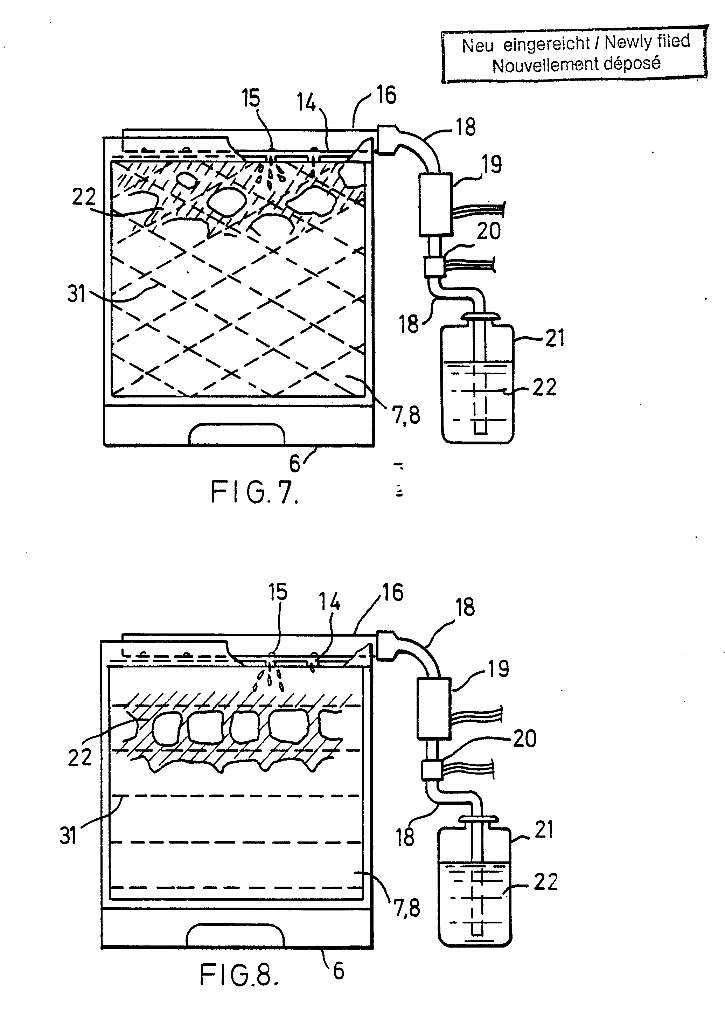

Although a deodorizing filter is used in the embodiment described above, an embodiment in which a plurality of filters are used to form laminated filters by sewing, whereby the deodorant sprayed can be equally distributed in the filter is shown in Figs. 7 and 8. The deodorant which has been sprayed are distributed along the seam 31, whereby further high deodorizing effect can be thus obtained, furthermore, overflow of the deodorant through the lower surface of the filter can be prevented. By addition of antibacterial agent to the deodorant, it is capable of keeping from getting moldy if the deodorant is remained to be wet, and it·is effective to keep rooms antibacterial condition.

Since the device according to the present invention is constituted so as to optionally or periodically spray the deodorizing filter with the deodorant, the deodorant·can be applied by way of the pump at any desired time wanted such as the removal of the offensive odor is needed, at the time in which the deodorizing effect of the deodorant in the filter deteriorates or when the deodorizing effect is intended to improve. The initial deodorizing effect can be kept without any concern to the state of the dust collected by the filter, and the numbers of needed change of filter can be reduced, consequently labor and cost can be kept small. By providing a seam for the filter, the deodorant can be destributed equally, whereby greater deodorizing effect can be obtained.

A spraying pipe 16 and a spraying hole 15 which are capable of preventing unequal distribution of the deodorant 22 which has been sprayed over the aforesaid filter 8 will now be described.

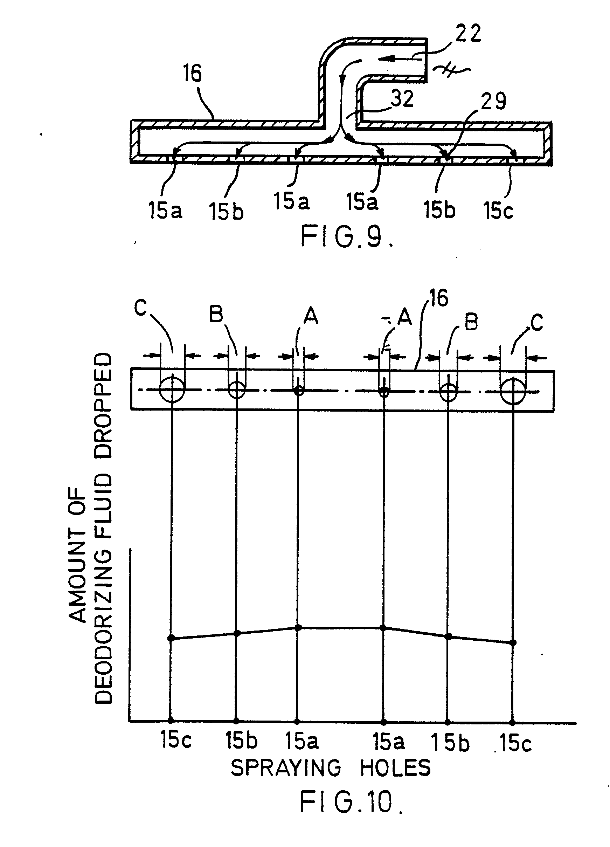

Fig. 9 is a partial cross sectional view illustrating a spraying pipe 16, in which reference numeral 16 represents a spraying pipe, reference numeral 32 represents a deodorant 22 supplying portion which is disposed for this pipe 16, reference numerals 15a, 15b and 15c represent spraying holes which are disposed in the spraying pipe 16 by which deodorant 22 which has been supplied from the supplying portion 32 is dropped to the deodorizing filter 8. These spraying holes 15a, 15b and 15c are provided a pulrality of, in Fig. 9, for example, a first spraying hole 15a, second spraying hole 15b and third spraying hole 15c are in sequence in accordance iwth the distance from the supplying portion 32. The diameters of the first spraying hole 15a, second spraying hole 15b and the third spraying hole 15c are arranged in sequence as A<B<C.

The function will now be described.

The diamters A, B and C of the aforesaid spraying holes 15a, 15b and 15c are arranged to be in sequence enlarged from the supplying portion 32 by distance thereof. That is, the diameter at the hole in the portion at which the supplying pressure of the deodorant from the supplying portion is large is small, where the pressure is small, the diameter is made large.

The deodorant 22 which has been ˙supplied through the supplying portion 32 can be dropped onto the deodorizing filter 18 through each one of spraying holes 15a, 15b and 15c by substantially equal quantity.

As a result of this, the deodorant 22 is equally distributed onto the deodorizing filter 8, whereby air flow containing offensive odor passing the deodorizing filter 8 can be applied to this deodorant 22 without exception, whereby deodorizing can be realized.

Although the embodiment shown in Fig. 9 has the supplying portion thereof at substantially center of the ˙spraying pipe, the supplying portion may be disposed at the end portion of it, as shown in Fig. 4, it can display same effect by way of arranging the diameters of the spraying holes to increase from the position near the supplying portion.

As mentioned above, and as shown in Fig. 9, since the diameters of the spraying holes for the deodorant are arranged to increase from the supplying portion in accordance with the distance from there, whereby quantity of the deodorant dropped through each spraying hole can be made uniform, therefore, the distribution of the deodorant over the filter can be made equal, consequently the deodorizing effect can be improved.

And only by disposing the supplying portion 32 adjacent to a center of the spraying pipe 16, as shown in Fig. 9, unequal distribution of the deodorant 22 can be reduced in comparison to that shown in the case in which the supplying portion 32 is disposed with the spraying pipe 16 as shown in Fig. 4.

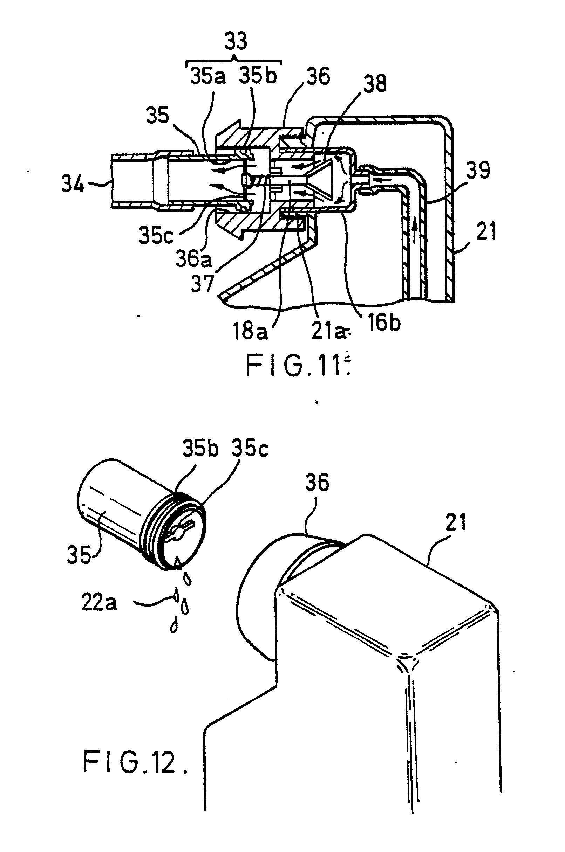

The connecting portion 33 in the case where this connecting portion 33 is positioned between the aforesaid deodorant chamber 21 and the intaking port of the spraying pump 19 will now be described with reference to Figs. 11 and 12.

The connecting portion 33 comprises a connecting pipe 35 one end of which is connected to a pipe 34 which is connected to the intaking port of the spraying pump 19 and a connection end 35a which is secured to a cap 36 which is screw fitted to a port 21a of the deodorant chamber 21. An 'O' ring 35b is secured to the outer circumference of the connection end 35a for the purpose of liquid tightness so as to be secured to a fastener D 36a of aforesaid cap 36.

A stopper 35c to which one end of a valve rod 38a of a closing valve 38 which is adapted to always move to pump side by means of a spring 37 is abutted is provided for the connection end 35a of the connecting pipe 35. A deodorant 22 supplying pipe disposed from the deodorant 22 inletting pipe 39 to the spraying pump 19 can be closed by way of contact of the closing valve 38 with the valve seat 36b provided on the inside of the cap 36.

The connecting pipe 35 constituted as shown in Figs. 11 and 12 raises a problem wherein the deodorant 22 which has been retained in the portion between the spraying pump 19 and the connecting portion 33 overflows through the end portion of the connecting pipe 35 forming a drop 22a as shown in Fig. 12 when the deodorant chamber 21 is detached for the purpose of supplying the deodorant, the inside of the air cleaner therefore made dirty, and if the quantity is large, the deodorant leaks outside of the air cleaner, which is followed by making the wall or floor made dirty.

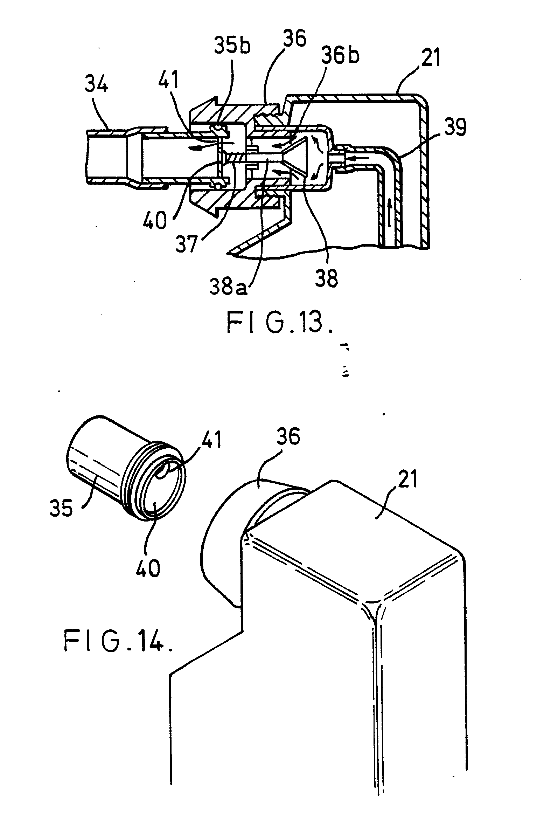

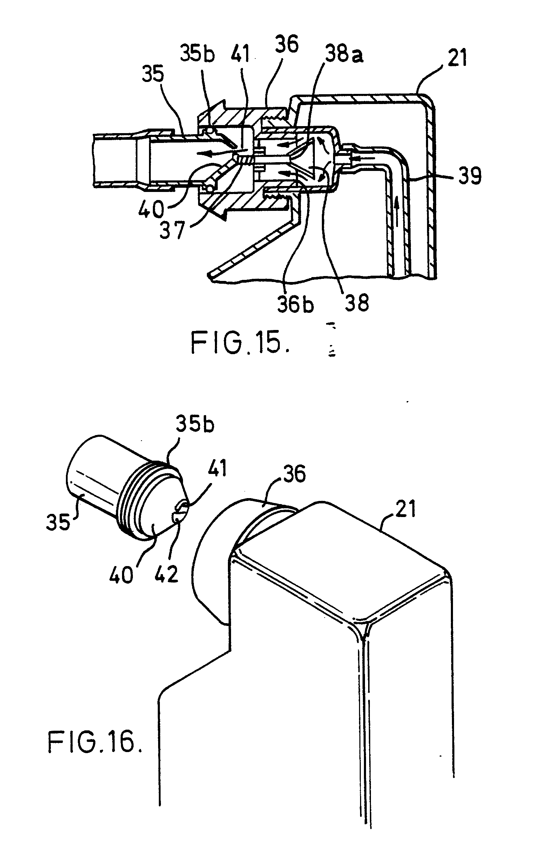

In order to prevent the deodorant remained between the spraying pump 19 and the connecting pipe from leakage when the deodorant chamber 21 is detached, a connecting pipe constituted in such a manner that the connecting pipe closes the opening port of the end portion and the connecting port is arranged facing upward is shown in Figs. 13 and 16.

Fig. 13 is a side cross sectional view illustrating the state wherein the deodorant chamber 21 and the connecting pipe 35 of the connecting portion 33 are connected by way of securing. Fig. 14 is a perspective view illustrating the state in which the connecting pipe 35 is detached from the deodorant chamber 21.

As illustrated in aforesaid drawings, a closing wall 40 is provided for the opening port of the connecting pipe 35 adjacent to the deodorant chamber 21, one end of a valve rod 38a of the closing valve 38 is abutted against this closing wall 40 by means of a spring 37, whereby a supplying pipe passage of the deodorant is disposed in a separated manner from the valve seat 36b of the cap 36.

An opening portion of the connecting pipe 35 adjacent to the deodorant chamber 21 is closed by way of provision of a connecting port 41 through which the deodorant 22 passes in the upper portion of this closing wall 40.

The operation of the connecting portion 33 shown in Figs. 13 and 14 will now be described.

A closing valve 38 which is provided for a cap 36 which forms the connecting portion 33 is pushed by a spring 37, whereby the closing valve 38 is brought into contact with the valve seat 36b, as a result of which the passage is closed. In this state, it is mounted onto the air cleaner for use. That is, since the closing wall 40 of the connecting pipe 35 is secured by pushing the valve rod 38a against the spring 37, the closing valve 38 which has been brought into contact with the valve seat 36a is separated, whereby the passage is opened.

When the fan motor 2 is turned on and the spraying pump 19 is also turned on in this state, the deodorant 22 is sucked from the deodorant chamber 21 by means of a spraying pump 19 through the suction pipe 39, whereby it is supplied through the opening port of the closing valve 38 and the connecting pipe 35 and the spraying hole 15 of the spraying pipe 16 onto the deodorizing filter 8. When the connecting pipe 35 of the connecting portion 33 is detached from the cap 36 of the deodorant chamber 21, the liquid deodorant in the connecting pipe 35 is not overflown since the connecting port 41 of the closing wall 40 of the connecting pipe 35 is bored only in the upper portion.

In Figs. 13 and 14, an embodiment in which the closing wall 40 of the connecting pipe 35 is formed in a flat shape is described, the closing wall 40 may be formed in a tapered shape as shown in Figs. 15 and 16, and a contacing surface 42 which acts as a stopper of the valve rod 38a may be provided at the top, above which the connecting port 42 may be provied.

As shown in Figs. 13 to 16, by closing the opening surface of the connecting pipe of the connecting portion between the deodorant chamber and the spraying pump adjacent to the deodorant chamber by a closing wall, and a connecting port is bored in the upper portion of this closing wall, the spilling of deodorant through the edge of the connecting pipe when the deodorant chamber is removed can be pevented, and further the connecting port of the connecting pipe is made small, whereby the flow rate become fast and intaking noise can be kept small by way of an oriffice effect.

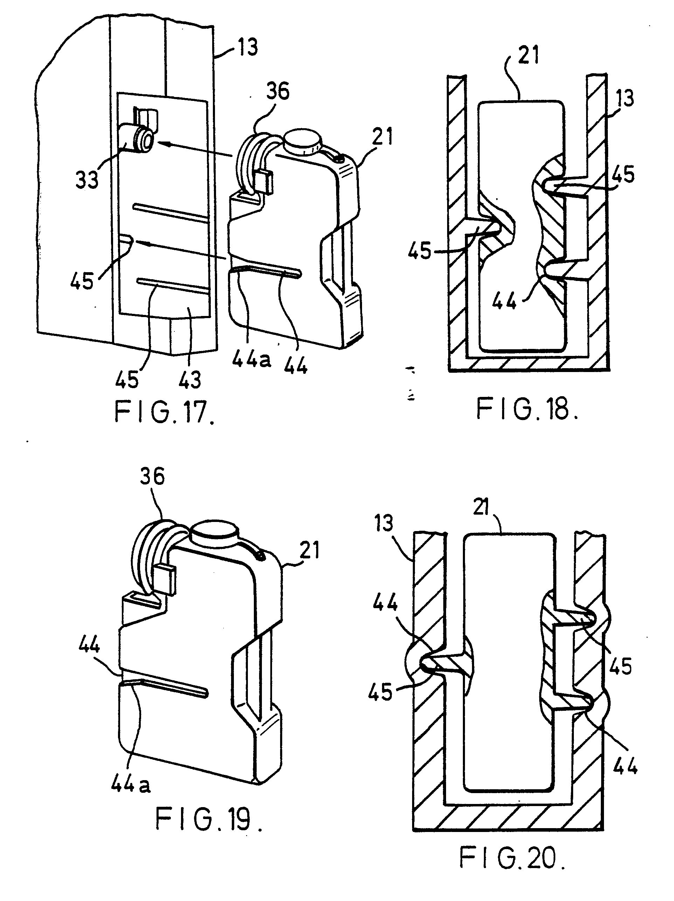

Finally, the structure for simply and securely mounting the deodorant chamber 21 into the main body of the air cleaner will now be described with reference to Figs. 17 to 20.

Fig. 17 is a cutaway perspective view illustrating an essential portion for the function of accommodating the deodorant chamber 21 in the deodorant chamber accommodating portion 43 in the casing 13 of the air cleaner 13.

In order to properly securing the connecting portion 33 which is fastened by means of a screw or the like into the casing 13 and a cap which is disposed in the deodorant chamber 21, a grooved recess 44 is formed in the side surface of the deodorant chamber 21 so as to act as a guide, and a rib shaped projection 45 of which shape corresponds to the former is provided on the inner surface of the deodorant chamber accommodating portion 43 of the casing 13, whereby they are secured for the purpose of properly guiding the cap 36 to the connecting portion 33.

Fig. 18 is a cross sectional view illustrating an essential portion for the state in which the deodorant chamber 21 is accommodated in the casing 13 in which the projecting portion 45 which is disposed on the inner surface of the casing 13 and the recess portion 44 which is provided in the side surface of the deodorant chamber 21 are secured each other.

Fig. 19 is a perspective view illustrating the deodorant chamber 21 in which reference numeral 44a represents a portion which is disposed at the entrance of the recess portion 44, and which acts to guide the corresponding projecting portion 45 of the rib.

When a user mounts the deodorant chamber 21 onto the deodorant chamber accommodating portion 43, as shown in Fig. 17, the deodorant chamber 21 is moved from the opening port of the accommodating portion 43 in the direction designated by an arrow. The projecting portion 45 which is disposed on the inner surface of the casing 13 is therefore brought into contact with the tapered portion 44a of the recessed portion 44 which is provided in the side surface of the deodorant chamber 21. It is properly guided from this tapered portion 44a to the recessed portion 44, the connecting portion 33 which is disposed in the casing 13 and the cap 36 effectively act as guides to secure them at a suitable position.

Although the case in which the projecting portion is provided on the casing side and the recessed portion on the chamber side is described in Figs. 17 to 19, the recessed portion 44, of course, may be provided on the casing side 13 and the corresponding shaped projecting portion 45 disposed on the deodorant chamber 21 side, whereby the similar effect can be displayed.

As mentioned above, as shown in Figs. 17 to 20, setting of the casing side connecting portion and the cap on the chamber side can be properly and accurately carried out when the deodorant chamber is mounted into the main body of the air cleaner in a simple and cheap manner securely, furthermore, suction of air and fluid leakage and so forth can be prevented.

## Claims

1. An air cleaner comprising a dust collecting filter (7), a deodorizing filter (8) and a fan (1) for generating a flow of air through the filters, characterised in that the air cleaner is provided with a source (21) of deodorant liquid, and means (16, 19) for distributing deodorant liquid from the source over the deodarizing filter (8).

2. An air cleaner comprising, in a main body, a dust collecting filter (7); a deodorizing filter (8); and a fan (1); characterised in that it further includes a deodorant chamber (21) which accommodates deodorant (22); a spraying pipe (16) disposed above said deodorizing filter (8) and having a plurality of spraying holes (15); a spraying pump (19) for supplying said spraying pipe with said deodorant in said deodorant chamber; and control means which control the driving of said spraying pump.

3. An air cleaner according to claim 2, wherein a supplying portion of said spraying pipe which is connected to the spraying pump side is positioned in the vicinity of the center of said spraying pipe.

4. An air cleaner according to claim 2 or 3, wherein diameters of said plurality of spraying holes of said spraying pipe are arranged to increase as the respective distance between each of said spraying holes and said supplying portion connected to said spraying pump side increases.

5. An air cleaner according to claim 1, 2, 3 or 4 wherein a closing wall is provided on the deodorant chamber side of a connecting pipe which connects said spraying pump and said deodorant chamber, and a connecting port is provided above said closing wall.

6. An air cleaner according to claim 1, 2, 3 or 4 wherein a tapered closing wall is provided on the deodorant chamber side of a connecting pipe which connects said spraying pump and said deodorant chamber, and a connecting port is provided at the top of said tapered portion.

7. An air cleaner according to claim 1, 2, 3 or 4 wherein a projecting portion or a recessed portion, is provided on the side surface of said deodorant chamber, and a corresponding recessed portion or projecting portion is provided on a side surface of the main body which accommodates said deodorant chamber, opposite said side surface of said deodorant chamber and so as to fit each other.

8. An air cleaner according to any preceding claim, wherein a part of said dust collecting filter is arranged to be a deodorizing filter.

9. An air cleaner according to any preceding claim, wherein a seam is provided in said deodorizing filter.

10. An air cleaner according to any preceding claim, wherein an antibacterial agent is mixed in said deodorant.

F I G . 1 .

F I G . 2 .

F I G . 3 .

FIG.4.

FIG.5.

FIG.6.

FIG.7.

FIG.8.

FIG.9.

FIG.10.

AMOUNT OF
DEODORIZING FLUID DROPPED

SPRAYING HOLES

FIG.11.

FIG.12.

FIG.13.

FIG.14.

FIG.15.

FIG.16.

FIG.17.

FIG.18.

FIG.19.

FIG.20.